# FASCICULE DE BREVET EUROPEEN

(11) **EP 2 729 076 B1**
(45) Date de publication et mention de la délivrance du brevet: **30.09.2015**
(21) Numéro de dépôt: 12738527.6
(22) Date de dépôt: 26.06.2012
(51) Int. Cl.: A61B 17/064, A61B 17/068, A61B 17/10

(54) **DISPOSITIF COMPRENANT UNE PLURALITE D'IMPLANTS POUR LA FIXATION DE MATERIEL PROTHETIQUE**
VORRICHTUNG MIT MEHREREN IMPLANTATEN ZUR BEFESTIGUNG VON PROTHESENMATERIAL
DEVICE COMPRISING A PLURALITY OF IMPLANTS FOR THE FIXING OF PROSTHETIC MATERIAL

(30) Priorité: 07.07.2011 FR 1156137; 17.01.2012 FR 1250451
(43) Date de publication de la demande: 14.05.2014
(73) Titulaire: Aspide Medical, 42350 La Talaudiere (FR)
(72) Inventeur: POMPEE, Christian, F-38410 Saint Martin d'Uriage (FR); CARTERON, Patrick, F-42600 Chalain Le Comtal (FR); WIECEK, William, F-42160 Bonson (FR)
(74) Mandataire: Cabinet Laurent & Charras
(86) Numéro de dépôt international: PCT/FR2012/051466
(87) Numéro de publication internationale: WO 2013/004947

(56) Documents cités:
- EP-A2- 2 389 873
- WO-A1-2007/098512
- FR-A1- 2 941 144
- US-A1- 2004 034 375
- US-A1- 2004 230 208
- US-A1- 2006 282 085
- US-A1- 2006 282 101
- US-A1- 2007 038 220

## Description

L'invention se rattache au secteur technique des implants utilisés pour assurer la fixation de matériel prothétique du type prothèse (renforts de parois) aux tissus mous et permettre le rapprochement de tissus mous.

L'invention concerne plus particulièrement les implants ou agrafes utilisés dans la fixation de prothèses dans le traitement des hernies abdominales pour la réparation des tissus mous, la reconstruction de parois abdominales.

L'utilisation et la pose d'implants ou agrafes s'effectuent à l'aide de pistolets ayant des chargeurs remplis d'une pluralité d'implants ou agrafes qui sont tous indépendants les uns des autres et qui sont distribués par une action de commande sur la détente du pistolet.

La problématique réside dans le fait que les implants sont guidés de manière plus ou moins aléatoire depuis le chargeur jusqu'au tube ou canon du pistolet destiné à venir en contact avec la prothèse à travers un trocart facilitant la mise en place par coelioscopie. En pratique, cela se traduit par un désalignement des implants successifs ce qui entraîne des bourrages et mise hors service du pistolet.

Différents systèmes de dispositifs existent, par exemple ceux décrits dans les brevets US 2006/0 129 154, US 7 758 612, US 5 431 669, US 5 904 693, US 5 743 880, US 5 282 808, WO 93/24059, EP 1 317 213, EP 1 237 484, WO 2007/123978, FR 2 841 765, US 2007/0 038 220 qui divulgue les caractéristiques du préambule de la présente revendication 1.

La démarche du Demandeur a donc été de réfléchir à une nouvelle manière de distribuer les implants à partir d'un pistolet spécifique, et ce, dans des conditions de fiabilité accrue, en évitant les risques de blocage du pistolet ou de sa mise hors service, ce qui pourrait être préjudiciable en situation d'intervention en salle opératoire.

La solution apportée par le Demandeur répond de manière nouvelle et inattendue à cette problématique.

Selon une première caractéristique de l'invention, le dispositif comprend une pluralité d'implants identiques disposés en alignement et liés les uns aux autres successivement par leur partie avant et leur partie arrière, de manière temporaire, et en ce que les dits implants ont une configuration extérieure en forme de vis, et en ce que chaque implant présente une cavité intérieure longitudinale de section triangulaire traversante totalement pour constituer le logement d'un moyen de pré-positionnement configuré selon une empreinte triangulaire avec une partie d'extrémité en pointe, et en ce que la liaison des implants entre eux par leurs faces en regard arrière et avant respectivement de deux implants successifs est une liaison temporaire, et en ce que la pluralité d'implants constitue un ensemble assemblé destiné à être introduit dans un bloc chargeur associé à l'ancillaire de pose.

Selon une autre caractéristique, l'ensemble des implants solidaires les uns aux autres est embroché sur le moyen de pré-positionnement configuré selon une section transversale triangulaire en vue de leur mise en place dans le tube chargeur du pistolet.

Selon une autre caractéristique, chaque implant présente une partie avant sans tête avec une face frontale transversale apparente discale et une partie arrière avec une face arrière transversale discale, les implants étant solidarisés lors de leur fabrication, et étant sécables les uns par rapport aux autres.

Selon une autre caractéristique, chaque implant présente une partie avant ayant une configuration d'assemblage mâle, semi-circulaire en forme de cran en prolongement de la partie intermédiaire formant corps de l'implant et une partie arrière avec une configuration d'assemblage femelle semi-circulaire en forme de cran en prolongement de la partie intermédiaire formant corps de l'implant, les dites parties d'assemblage formant crans mâle-femelle, s'emboîtant l'une dans l'autre pour constituer un assemblage d'implants successifs en permettant un positionnement précis dans le bloc chargeur par le dit moyen de pré-positionnement.

Selon une autre caractéristique, le bloc chargeur d'implants est tubulaire et reçoit intérieurement un guide sous forme de manchon ayant un profil à pas de vis régulier dont le profil est défini pour autoriser la réception de la pluralité d'implants constituant un tout monobloc, et leur rotation et avancement dans le bloc par l'actionnement en rotation du moyen de pré-positionnement, le dit guide étant à position fixe dans le bloc chargeur.

Selon une autre caractéristique, la mise en oeuvre et le positionnement des implants successivement sur la prothèse à fixer s'effectuent en présentant le bloc chargeur rempli de la pluralité d'implants solidarisés entre eux par le biais d'un moyen de pré-positionnement en regard de la prothèse à fixer, à présenter l'extrémité pointue du dit moyen de pré-positionnement à configuration triangulaire en égard de la prothèse et par rotation à permettre la fixation de l'implant immédiat sur la prothèse, puis à retirer en mouvement arrière le dit moyen de pré-positionnement lequel agit en contre-poussée sur l'implant suivant celui qui a été posé de manière à créer une force de poussée suffisante pour détacher et séparer l'implant posé de celui immédiat adjacent à l'encontre de la force de retenue générée par la fixation de l'implant posé sur la prothèse, par désolidarisation de leurs parois initiales de liaison.

Ces caractéristiques et d'autres encore ressortiront bien de la suite de la description.

Pour fixer l'objet de l'invention illustré d'une manière non limitative illustré aux figures des dessins où :
La figure 1 est une vue d'un implant seul selon l'invention, dans une première variante de réalisation,
La figure 2 est une vue d'un ensemble d'implants liés entre eux formant un tout homogène, selon la configuration de la figure 1,
La figure 3 est une vue à caractère schématique illustrant l'ensemble d'implants selon l'invention selon les figures 1 et 2 disposé dans un tube chargeur associé à un pistolet, l'ensemble des implants se trouvant dans le tube chargeur,
La figure 4 est une vue selon la figure 3 montrant la sortie partielle d'un implant du dit chargeur,
La figure 5 est une vue selon la figure 4 illustrant la sortie complète d'un implant,
La figure 6 est une vue selon la figure 5 montrant la séparation complète d'un implant par rapport à l'ensemble du bloc d'implant et retrait du moyen de pré-positionnement,
Les figures 7A, 7B, 7C, 7D sont des vues à grande échelle, en coupe, illustrant le bloc chargeur d'implant recevant dans sa version optimisée un guide profilé à pas de vis régulier susceptible de recevoir et de guider l'ensemble du bloc d'implants, les différentes vues représentant comme aux figures 3 à 6 le positionnement et l'avancement du bloc d'implants en vue d'une séparation de celui en position avant,
La figure 7E est une vue de face selon la figure 7A du bloc chargeur d'implants incluant le guide,
La figure 8 est une vue en perspective d'un ensemble de deux implants destinés à être assemblés dans une seconde variante de réalisation,
La figure 9 est une vue en perspective d'une pluralité d'implants mis en oeuvre selon la figure 8 et assemblés avec une coupe partielle longitudinale illustrant leur positionnement respectif,
La figure 10 est une vue à caractère schématique illustrant la mise en place d'une pluralité d'implants selon la figure 8, dans un bloc chargeur, lui-même inséré dans le conduit tubulaire d'un outil de pose,
La figure 11 est une vue d'un ensemble d'implants établi selon la figure 8 et introduit dans un outil de pose constitué par un tournevis.

Afin de rendre plus concret l'objet de l'invention, on le décrit maintenant d'une manière non limitative illustrée aux figures des dessins.

Le dispositif selon l'invention est référencé dans son ensemble par (D). Il comprend une pluralité d'implants (1) identiques disposés en alignement axial et liés les uns aux autres pour constituer par exemple un bloc de 10 à 30 implants pouvant être monté dans un bloc chargeur tubulaire d'implants associé à un outil de pose pouvant être un ancillaire du type pistolet, ou un tournevis. Les dits implants sont liés par leur partie avant (1a) et leur partie arrière (1b) de manière temporaire puis mis en place dans un bloc chargeur ayant une configuration tubulaire (2) destiné à être positionné sur un moyen de pré-positionnement. Le bloc chargeur est associé de toute manière appropriée à l'ancillaire de pose.

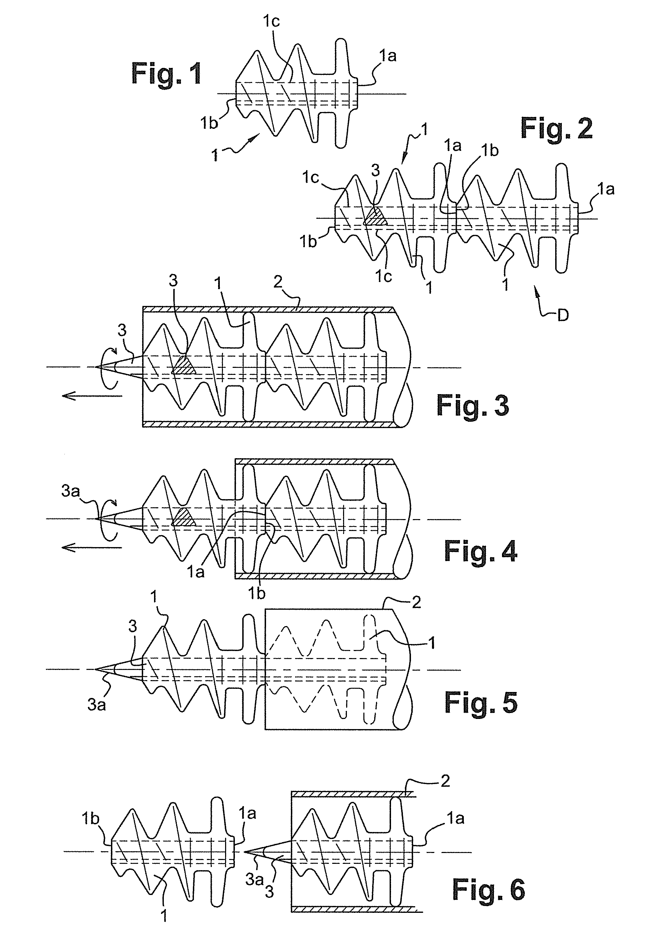

Selon une première réalisation illustrée aux figures 1 à 7, les dits implants sont liés par leur partie arrière et par leur partie avant lors de leur fabrication. Ces dits implants sont sécables les uns par rapport aux autres et ils sont rendus solidaires lors de leur fabrication avec une configuration de liaison temporaire. Cette liaison peut être une liaison moléculaire, ou par des lignes ou zones d'affaiblissement selon le mode de fabrication. Les implants peuvent ainsi être obtenus par moulage. Les implants (1) ont une configuration extérieure en forme de vis. Leur partie avant (1a) est sans tête avec une face frontale transversale apparente discale et une partie arrière (1b) avec une face transversale, discale. Intérieurement, chaque implant présente dans son corps (1d) une cavité intérieure (1c) longitudinale de configuration triangulaire et traversant totalement l'implant de sa face avant à sa face arrière. Cette cavité constitue le logement d'un moyen de pré-positionnement (3) configuré dans une section avec empreinte triangulaire et extrémité débordante (3a) pointue. Ainsi, le dit moyen de positionnement (3) permet l'embrochement successif d'une pluralité d'implants solidarisés entre eux. Ce moyen de pré-positionnement (3) est lui-même soumis à un effet de rotation pour permettre l'avancement progressif des implants et ce par un moyen de commande approprié disposé dans le pistolet.

Il convient dès lors d'exposer le procédé de pose des implants dans cette première variante de réalisation.

Préalablement, un bloc d'implants solidarisés entre eux est embroché par le moyen de pré-positionnement (3) en étant introduit dans le tube (2) associé au chargeur du pistolet. On présente le tube chargeur avec ou sans trocart en fonction des applications en regard de la prothèse à fixer et on présente l'extrémité pointue du dit moyen (3) sur la prothèse. On actionne ensuite le moyen (3) en rotation pour permettre le vissage de l'implant sur la prothèse. Comme représenté aux figures 4 et 5, l'implant situé en avant du bloc d'implants est progressivement sorti du bloc chargeur (2) pour être fixé sur la prothèse. Pour assurer la séparation de l'implant posé du reste du bloc, on procède alors au retrait du moyen (3). Celui- ci provoque une force de poussée, qui à l'encontre de la force de retenue générée par la fixation de l'implant posé sur la prothèse, provoque la séparation de l'implant posé par rapport au reste du bloc d'implants par désolidarisation de leurs parois initiales de liaison. La mise en rotation du moyen (3), et donc des implants et son avancement ou retrait, s'effectue de toute manière appropriée. Les implants sont réalisés en matériaux bio-résorbables, ou en matériaux non résorbables, tout en étant compatibles avec leur environnement de posologie bien connu de l'Homme des Métiers.

En fonction des conditions d'application, par exemple, pour les techniques de laparoscopie et laparotomie, l'ancillaire peut être réalisé sous forme de pistolet, à usage unique, ou à usage multiples re-stérilisable, ou le cas échéant, sous forme de tournevis.

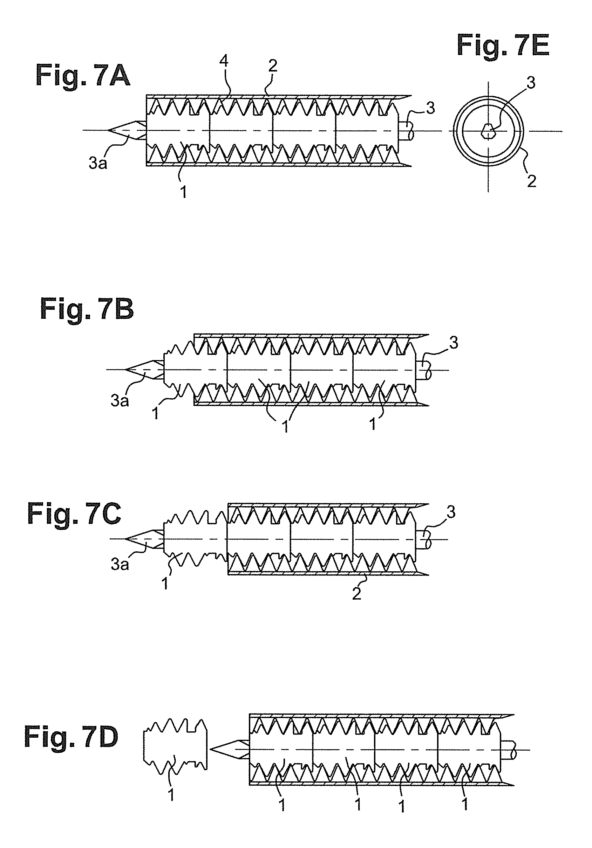

Dans une mise en oeuvre optimisée représentée aux figures 7A à 7E, le bloc chargeur (2) d'implants (1), qui assure notamment la protection extérieure des implants reçoit intérieurement un guide (4) en forme de manchon de grande longueur correspondant en tout ou partie à celle du bloc chargeur et présentant un profil de vis à pas régulier. Ce profil est défini pour autoriser la réception de la pluralité d'implants (1) constituant un tout monobloc en assurant un guidage des dits implants en rotation et en avancement ou recul dans le guide, à la manière d'un écrou, par l'actionnement du moyen (3) de pré-positionnement. Le profil à pas régulier du guide est donc adapté au profil en vis des implants. Le guide est à position fixe dans le bloc chargeur.

La solution technique selon l'invention permet de réaliser en quelque sorte des 'brochettes' d'une pluralité d'implants ou agrafes qui sont parfaitement guidées de par leur liaison respective dans le tube du chargeur associé au pistolet. Il n'y a aucun risque à ce que les implants puissent se désolidariser les uns des autres tant qu'ils sont dans la position à l'intérieur du tube du chargeur. Le pistolet peut donc être utilisé dans des opérations continues sans risque de nuisance.

Le nombre d'implants embrochés peut varier selon les besoins et la configuration du pistolet et ce en fonction des applications.

La liaison des implants entre eux est avantageusement moléculaire ou avec des zones d'affaiblissement, mais toute autre forme de liaison peut être envisagée, l'essentiel étant qu'elle soit temporaire et qu'elle puisse être facilement détruite après la pose d'un implant et retrait du bloc suivant.

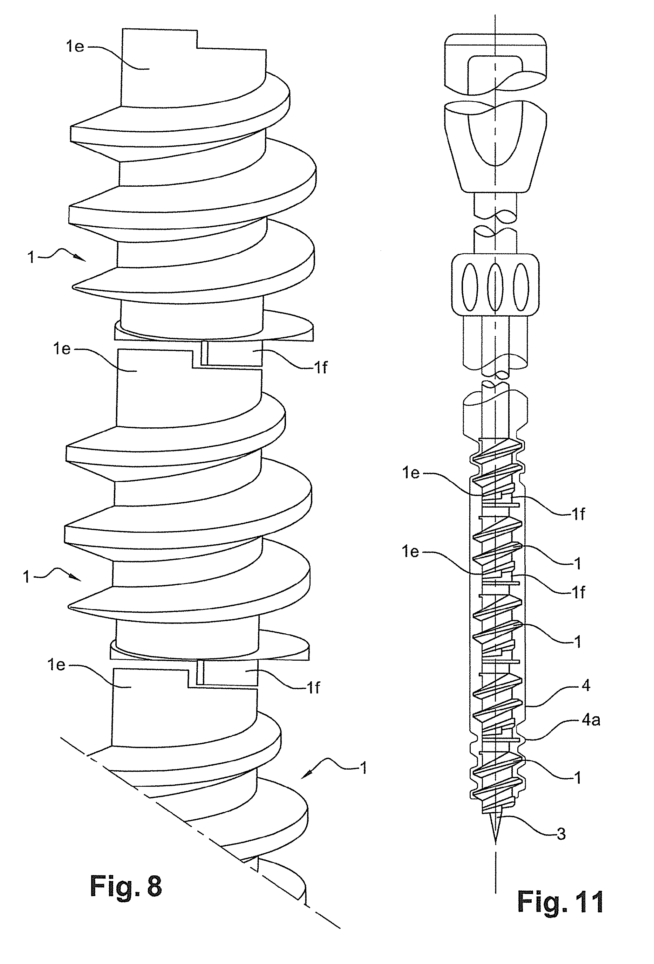

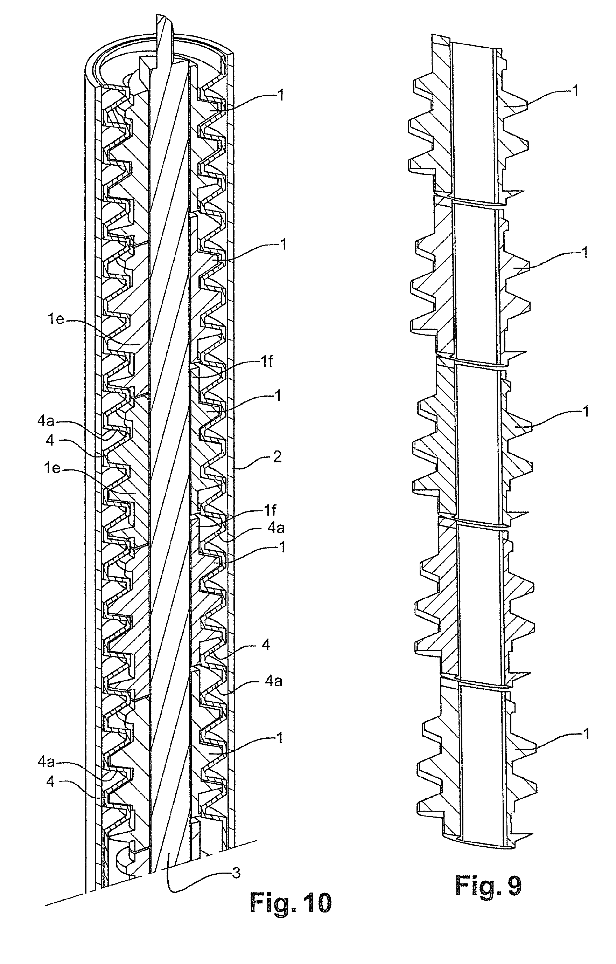

Dans la variante illustrée aux figures 8 à 11, chaque implant (1) présente la même configuration intérieure avec la cavité (1c) de configuration triangulaire traversant la totalité de chaque implant. Par contre, chaque implant présente une partie avant avec une configuration d'assemblage mâle (1e) en hélice, semi-circulaire, en formant cran en prolongement de la partie intermédiaire du corps (1d), et une partie arrière (1b) en configuration d'assemblage femelle (1f) en hélice, formant cran, semi-circulaire et en prolongement de la dite partie intermédiaire du corps (1d). Avantageusement, les parties avant (1a) et (1b) sont en section transversale oblique. De par la configuration en hélice liée avec la configuration extérieure en forme de vis de l'implant, les deux parties d'assemblage mâle (1e) et femelle (1f) en forme de crans sont complémentaires et s'assemblent par emboîtement entre elles pour constituer un assemblage de deux implants consécutifs.

Selon cette mise en oeuvre, de par la liaison obtenue, on obtient un entraînement en rotation des implants successifs lors de la manipulation du moyen (3) de pré-positionnement.

A cet effet, et selon une disposition importante, le guidage des implants (1) dans les deux variantes de réalisation des figures 1 à 7 et 8 à 11 est obtenu par la configuration du bloc chargeur (4) récepteur qui est tubulaire mais qui présente un refoulage (4a) par l'intérieur en configuration de pas de vis pour guider les dits implants et permettre leur avancement.

Les dits implants (1) peuvent être distribués par un outil connu dans la technique, à savoir un pistolet, mais également, par un tournevis comme représenté figure 11.

Dans la configuration des figures 8 à 11, les implants (1) ainsi assemblés par emboîtement se présentent dans une configuration en brochette sur le moyen (3) récepteur.

Les avantages ressortent bien de l'invention et on souligne le nouveau concept de réalisation des dits implants.

## Revendications

1. Dispositif comprenant une pluralité d'implants identiques pour la fixation de matériel prothétique, les dits implants ayant une configuration extérieure en forme de vis et étant disposés en alignement,
**caractérisé en ce que** les dits implants (1) sont liés les uns aux autres successivement par leur partie avant (1a) et leur partie arrière (1b), de manière temporaire, **et en ce que** chaque implant présente une cavité intérieure (1c) longitudinale de section triangulaire traversante totalement pour constituer le logement d'un moyen de pré-positionnement (3) configuré selon une empreinte triangulaire avec une partie d'extrémité (3a) en pointe, **et en ce que** la liaison des implants entre eux par leurs faces en regard arrière et avant respectivement de deux implants successifs est une liaison temporaire, **et en ce que** la pluralité d'implants constitue un ensemble assemblé destiné à être introduit dans un bloc chargeur associé à l'ancillaire de pose.

2. Dispositif selon la revendication 1, **caractérisé en ce que** l'ensemble des implants solidaires les uns aux autres est embroché sur le dit moyen de pré-positionnement (3) configuré selon une section transversale triangulaire en vue de leur mise en place dans le tube chargeur du pistolet.

3. Dispositif selon la revendication 1, **caractérisé en ce que** chaque implant présente une partie avant sans tête avec une face frontale transversale apparente discale et une partie arrière avec une face arrière transversale discale, les implants étant solidarisés lors de leur fabrication, et étant sécables les uns par rapport aux autres.

4. Dispositif selon la revendication 3, **caractérisé en ce que** les implants successifs sont solidarisés entre eux par une liaison moléculaire.

5. Dispositif selon la revendication 3, **caractérisé en ce que** les implants successifs sont solidarisés entre eux par des lignes ou zones d'affaiblissement.

6. Dispositif selon l'une des quelconques revendications 1 à 5, **caractérisé en ce que** les implants successifs sont réalisés par moulage.

7. Dispositif selon la revendication 1, **caractérisé en ce que** chaque implant présente une partie avant ayant une configuration d'assemblage mâle (1e), semi-circulaire en forme de cran en prolongement de la partie intermédiaire formant corps de l'implant et une partie arrière avec une configuration d'assemblage femelle (1f) semi-circulaire en forme de cran en prolongement de la partie intermédiaire formant corps de l'implant, les dites parties d'assemblage formant crans mâle-femelle, s'emboîtant l'une dans l'autre pour constituer un assemblage d'implants successifs en permettant un positionnement précis dans le bloc chargeur par le dit moyen de pré-positionnement.

8. Dispositif selon l'une des quelconques revendications 1 à 6, comprenant le dit bloc chargeur,
**caractérisé en ce que** le bloc chargeur est tubulaire et reçoit intérieurement un guide (4) sous forme de manchon ayant un profil à pas de vis régulier dont le profil est défini pour autoriser la réception de la pluralité d'implants constituant un tout monobloc, et leur rotation et avancement dans le bloc par l'actionnement en rotation du moyen de pré-positionnement (3), le dit guide étant à position fixe dans le bloc chargeur.

9. Dispositif selon l'une quelconque des revendications 1, 3 et 7, comprenant le dit bloc chargeur,
**caractérisé en ce que** le guidage des implants (1) est obtenu par la configuration du bloc chargeur (4) récepteur qui est tubulaire et qui présente un refoulage (4a) par l'intérieur en configuration de pas de vis pour guider les dits implants et permettre leur avancement.

## Patentansprüche

1. Vorrichtung, bestehend aus mehreren identischen Implantaten, zur Befestigung von Prothesen wobei die Implantate (1) außen über eine Gewindeform verfügen und hintereinander liegen, **dadurch gekennzeichnet dass** die Implantaten nach und nach am vorderen (1a) und hinteren (1b) Teil temporär miteinander verbunden sind und dass jedes Implantat im Zentrum ein inneren Hohlraum (1c) aufweist der mit einem dreieckigen Querschnitt in Längsrichtung innen entlang des ganzen Implantats verläuft zur Bildung einer Lagerung eines Ausrichtungsmittels (3) das ebenfalls dreieckig im Querschnitt ausgebildet ist und über ein spitzes Ende (3a) verfügt, und dass die Verbindung der Implantate untereinander, jeweils an den vorderen und hinteren gegenüberliegenden Seiten temporär ist, und dass die mehrere Implantaten eine Zusammengebaute Einheit bilden, welche dazu gedacht ist, in einen Blocklader eingeführt zu werden, der mit dem Hilfsgerät für den Einsatz der Prothese verbunden ist.

2. Vorrichtung nach Anspruch 1, **dadurch gekennzeichnet, dass** die miteinander verbundenen Implantate auf das Ausrichtungsmittel (3) aufgesteckt sind, dass ein länglich mit dreieckigem Querschnitt aufweist für deren Einsatz in das Laderohr der Pistole.

3. Vorrichtung nach Anspruch 1, **dadurch gekennzeichnet, dass** jedes Implantat über einen Vorderteil ohne Kopfstück verfügt, mit frei liegender transversalen, scheibenförmigen Vorderseite und einem hinteren, transversalen und scheibenförmigen Teil, wobei die Implantate bei der Herstellung miteinander verbunden werden und können voneinander getrennt werden.

4. Vorrichtung nach Anspruch 3, **dadurch gekennzeichnet, dass** die aufeinanderfolgenden Implantate molekular miteinander verbunden sind.

5. Vorrichtung nach Anspruch 3, **dadurch gekennzeichnet, dass** die Verbindungen zwischen den Implantaten Verschwächungszonen oder Verschwächungslinien darstellen.

6. Vorrichtung nach einem der Ansprüche 1 bis 5, **dadurch gekennzeichnet, dass** die aufeinanderfolgenden Implantaten gegossen wurden.

7. Vorrichtung nach Anspruch 1, **dadurch gekennzeichnet, dass** jedes Implantat eine vordere Seite aufweist die über eine männliche Steckverbindung (1e) verfügt, die halbkreisförmig, in Form einer Raste, in Verlängerung des Mittelteils der das Implantat selbst bildet, ausgebildet ist, und eine hintere Seite aufweist die über eine weibliche Steckverbindung (1f) verfügt, die halbkreisförmig, in Form einer Raste, in Verlängerung des Mittelteils der das Implantat selbst bildet ausgebildet ist, wobei die Verbindungsteile männliche und weibliche Rasten bilden die sich ineinander fügen zur Bildung einer Abfolge von Implantaten, wobei durch das Ausrichtemittel eine exakte Positionierung im Blocklader erreicht wird.

8. Vorrichtung nach einem der Ansprüche 1 bis 6 mit dem Blocklader, **dadurch gekennzeichnet, dass** der Blocklader rohrförmig ist, mit innenliegender Manschette zur Führung (4), wobei die Manschette über ein regelmäßiges Gewinde verfügt, dessen Konfiguration zur Aufnahme der Implantate die einen einzigen Block bilden, dient, sowie deren Rotation und Vorschub in dem Block, durch Drehen des Ausrichtemittels (3), wobei die Führung fest im Blocklader positioniert ist.

9. Vorrichtung nach einem der Ansprüche 1, 3 oder 7 mit Blocklader, **dadurch gekennzeichnet, dass** die Führung der Implantate (1) im Block durch die Konfiguration des aufnehmenden röhrenförmigen Blockladers (4) selbst gewährleistet wird und der innenliegend eine Knickstelle (4a) in Gewindesteigungskonfiguration zum Vorschub der Implantate.

## Claims

1. System comprising several identical implants for fixing a prosthetic equipment, the said implants having a screw type external configuration and arranged in a straight line, **characterised in that** the said implants (1) are connected to each other in succession by their front parts (1a) and rear parts (1b), on a temporary basis, **and in that** every implant has a longitudinal inner cavity (1c) with a triangular section that is completely penetrated to constitute the casing of a pre-positioning element (3) configured according to a triangular indentation with a pointed end part (3a), **and in that** the contact between the implants by their front and rear sides with two successive implants respectively is a temporary contact, **and in that** the multiple implants constitute an assembled unit that is designed to be implanted in a charging block related to the insertion ancillary.

2. The system according to claim 1, **characterised in that** all the implants connected to each other are skewed on the said pre-positioning element (3) configured as per a penetrated triangular section in order to position them in the filling pipe of the pistol.

3. The system according to claim 1, **characterised in that** every implant has a headless front part with a discal apparent transversal frontal side and a rear part with a discal transversal rear side, the implants being connected during their production, and can be separated with respect to the other implants.

4. The system according to claim 3, **characterised in that** the successive implants are connected to each other by a molecular bond.

5. The system according to claim 3, **characterised in that** the successive implants are connected to each other by weakening lines or areas.

6. The system according to one of the claims between 1 and 5 **characterised in that** the successive implants are made by moulding.

7. The system according to claim 1, **characterised in that** each implant has a front part with a semi-circular male assembly configuration (1e), in the shape of a notch extending the intermediate part that forms the body of the implant and a rear part with a semi-circular female assembly configuration (1f) in the shape of a notch extending the intermediate part that forms the body of the implant, the said assembly parts forming male-female notches, fitting one inside the other in order to constitute an assembly of successive implants while facilitating a precise positioning in the charging block by the said pre-positioning element.

8. The system according to one of the claims between 1 and 6, comprising the said charging block, **characterised in that** the charging block is tubular and internally accommodates a guide (4) in the form of a connecting sleeve with a regular screw thread profile wherein the profile is defined for allowing the reception of multiple implants constituting a single whole piece, and their rotation and penetration into the piece by actuation in rotation of the prepositioning element (3), the said guide being in a fixed position in the charging block.

9. The system according to one of the claims between 1, 3 and 7, comprising the said charging block, **characterised in that** the guiding of the implants (1) is obtained by the configuration of the receiving charging block (4) that is tubular and has a groove (4a) inside in a screw thread configuration in order to guide the said implants and enable their penetration.
